# EUROPEAN PATENT SPECIFICATION

(11) **EP 3 903 321 B1**
(45) Date of publication and mention of the grant of the patent: **05.08.2026**
(21) Application number: 20709455.8
(22) Date of filing: 11.02.2020
(51) Int. Cl.: G16H 40/20, G16H 20/40, A61M 1/14

(54) **SHARING DIALYSIS MACHINES**
GEMEINSAM GENUTZTE DIALYSEMASCHINEN
MISE EN COMMUN DE MACHINES DE DIALYSE

(30) Priority: 11.02.2019 US 201962803743 P
(43) Date of publication of application: 03.11.2021
(73) Proprietor: Fresenius Medical Care Holdings, Inc., Waltham, MA 02451-1457 (US); Fresenius Medical Care Deutschland GmbH, 61352 Bad Homburg (DE); Fresenius Medical Care AG, 61352 Bad Homburg (DE)
(72) Inventor: MERCHANT, Stephen A., Waltham, MA 02451-1457 (US); SCHERMEIER, Olaf, 61352 Bad Homburg (DE); KOULECHOV, Kirill, 61352 Bad Homburg (DE); CERMAN, Zdenek, 61352 Bad Homburg (DE); HAUKE, Christopher, 61352 Bad Homburg (DE)
(74) Representative: Fish & Richardson P.C.
(86) International application number: PCT/US2020/017596
(87) International publication number: WO 2020/167711

(56) References cited:
- EP-A1- 3 100 750
- US-A1- 2002 131 572
- US-A1- 2009 125 325
- US-A1- 2013 310 726
- US-A1- 2013 346 099
- US-A1- 2014 276 373
- US-A1- 2018 144 817

## Description

### TECHNICAL FIELD

This disclosure relates to dialysis sharing over a network of dialysis systems.

### BACKGROUND

Dialysis is a treatment used to support a patient with insufficient renal function. The two principal dialysis methods include hemodialysis (HD) and peritoneal dialysis (PD). During HD, the patient's blood is passed through a dialyzer of a dialysis machine, while a dialysis solution (or, dialysate) is also passed through the dialyzer. A semi-permeable membrane in the dialyzer separates the blood from the dialysate and allows fluid exchanges to take place between the dialysate and the blood stream via diffusion, osmosis, and convective flow. These exchanges across the membrane result in the removal of waste products (e.g., such as solutes, like urea and creatinine) from the blood. These exchanges also regulate the levels of other substances (e.g., sodium and water) in the blood. In this way, the dialysis machine acts as an artificial kidney for cleansing the blood.

During peritoneal dialysis ("PD"), the patient's peritoneal cavity is periodically infused with dialysate. The membranous lining of the patient's peritoneum acts as a natural semi-permeable membrane that allows diffusion and osmosis exchanges to take place between the solution and the blood stream. These exchanges across the patient's peritoneum result in the removal of waste products (e.g., such as solutes, like urea and creatinine) from the blood and regulate the levels of other substances (e.g., sodium and water) in the blood.

US 2009/0125325 describes a data processing system for assigning patients to providers of medical services and/or equipment. The system comprises a database of providers of medical services and equipment for patients, the database comprising records which comprise provider, medical service, medical equipment, instrumentation, location, and time slot data, means to receive a request for a medical procedure requiring a specific instrumentation for a first patient or for medical equipment, and means to return at least one time slot for the specific instrumentation for the medical procedure in response to the request.

US 2002/0131572 describes a method and system where a server system is able to handle a majority of the processing work in determining and booking available schedule appointments between a patient's schedule and the schedules of the required resources (e.g., personnel, examination rooms, equipment, services, etc.).

### SUMMARY

The invention is defined in the claims.

Claim 1 defines a method executed by a dialysis hub system.

In some examples, the method further includes transmitting, using the data processor, an alternate reservation to use a dialysis machine from the list of available dialysis machines.

In some examples, the method further includes transmitting, using the data processor, instructions for self-cannulation.

In some examples, the method further includes transmitting, using the data processor, dialysis information.

In some examples, the method further includes communicating, using the data processor, with emergency personnel.

In some examples, the reservation to use the dialysis machine is rescheduled at regular intervals.

In some examples, the method further includes receiving, using the data processor, diagnostic data for the dialysis machine.

Claim 8 defines a dialysis network system.

In some examples, the system further includes transmitting dialysis data to the client device.

In some examples, the system further includes transmitting dialysis data to a physician terminal.

In some examples, the system further includes communicating with an emergency terminal.

In some examples, the system further includes transmitting information from a database to the client device.

In some examples, the system further includes updating the database with information from the client device.

Claim 14 defines a non-transitory computer storage medium encoded with a computer program, the program comprising instructions that when executed by data processing apparatus cause the data processing apparatus to perform operations of the methods described above.

Other aspects, features, and advantages of the subject matter included herein will be apparent from the description and drawings, and from the claims.

### DESCRIPTION OF DRAWINGS

FIG. 1 is a perspective view of a fluid conditioning system that can cooperate with a dialysis system to carry out a fluid conditioning cycle that includes a dialysis treatment.
FIG. 2 is a top view of the fluid conditioning system of FIG. 1.
FIG. 3 is a front view of the fluid conditioning system of FIG. 1.
FIG. 4 is a rear view of the fluid conditioning system of FIG. 1.
FIG. 5 illustrates an example setup of the fluid conditioning system of FIG. 1.
FIG. 6 is a front perspective view of a hemodialysis system.
FIG. 7 shows an example of a peritoneal dialysis system.
FIG. 8 is an exemplary method for requesting use of a dialysis machine in a dialysis sharing network.
FIG. 9 is an exemplary system for a dialysis sharing network system.

### DETAILED DESCRIPTION

This document discusses sharing dialysis machines, and techniques for sharing dialysis machines. Sharing of dialysis machines can be controlled and monitored over a distributed network (e.g., the Internet) using a central hub. As discussed in more detail below, in some implementations, techniques include the use of a central hub that can be configured to communicate between multiples parties including but not limited to patients, providers of dialysis machines, medical personnel, and emergency personnel. The communications can include dialysis information such as diagnostic and treatment data, prescription data, patient identification and dialysis operation information between the parties.

Sharing of dialysis machines can allow for patients seeking dialysis services and providers owning dialysis machines to participate in a mutually-beneficial network. Sharing dialysis machines over a network allows patients to seek dialysis machines that better suite their needs (based at least partially on location and availability of dialysis machines, and provides comforts of a home service rather than a hospital setting), and allows providers to profit from the services provided related to their dialysis machines. Additionally, the cost for patients can be reduced via providers reducing their service prices to be competitive.

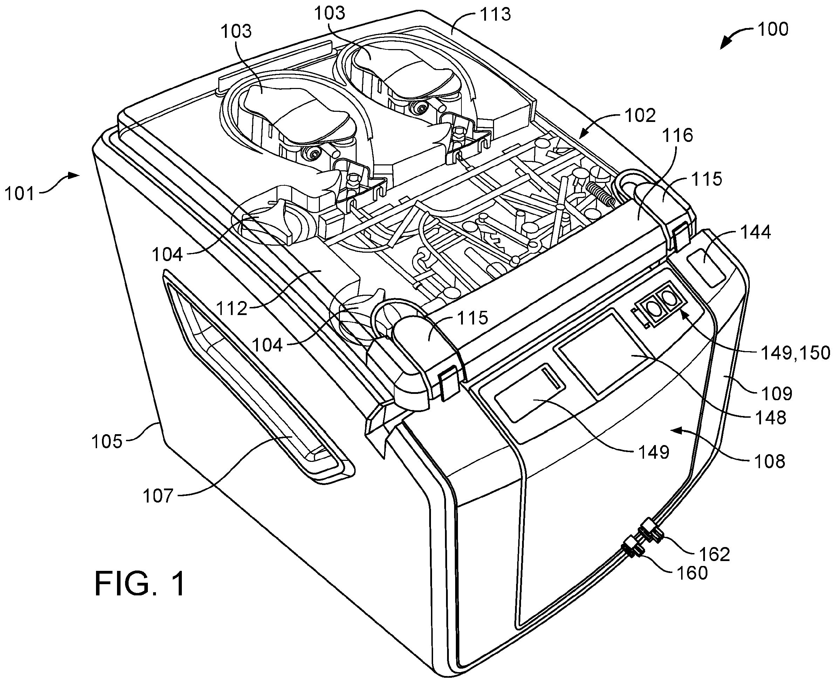

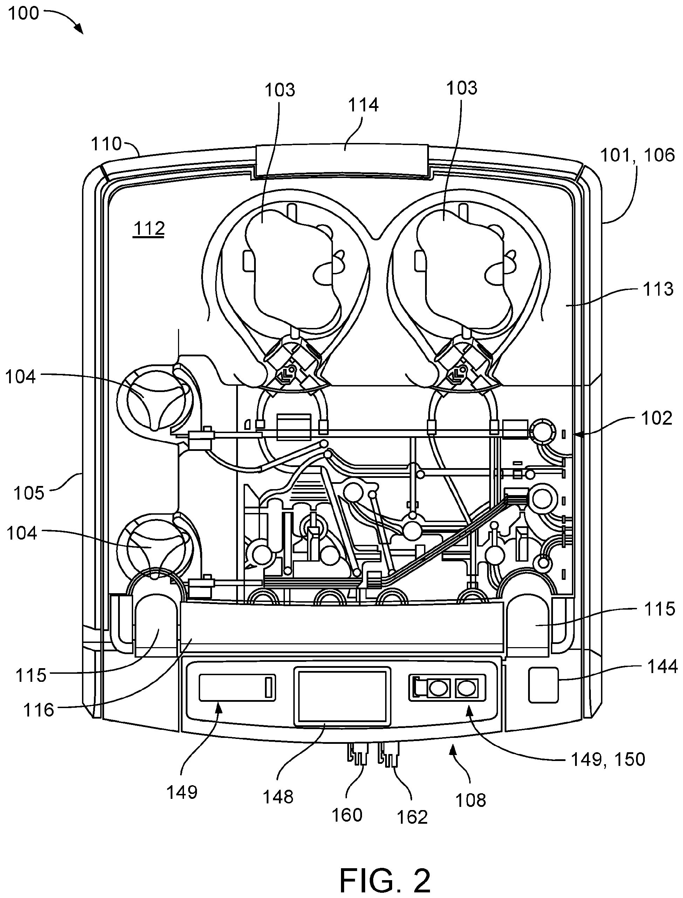

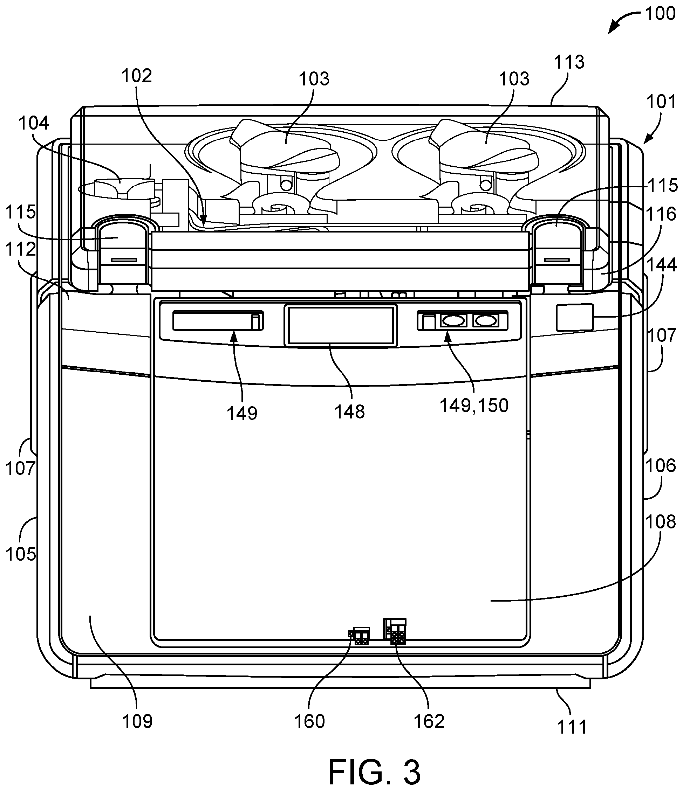

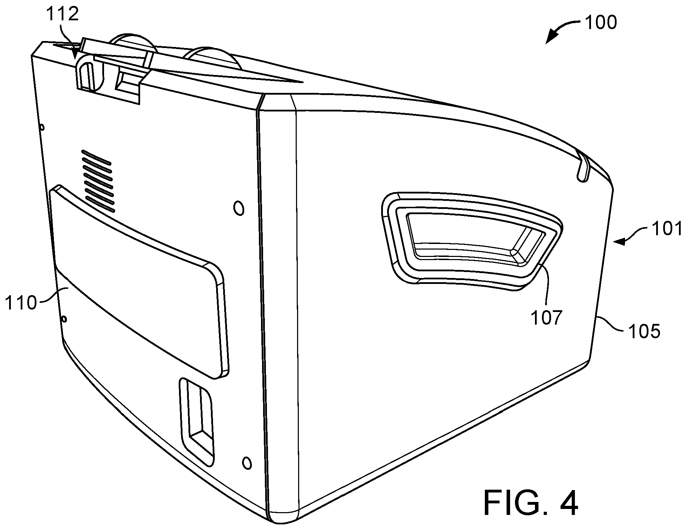

FIGS. 1-4 illustrate a fluid conditioning system 100 that can be operated to prepare conditioned dialysate for use in a dialysis system. For example, the fluid conditioning system 100 can be fluidly communicated with the dialysis system to deliver "fresh" (e.g., cleaned, conditioned) dialysate to the dialysis system, collect "spent" (e.g., contaminated, unconditioned) dialysate from the dialysis system, and regenerate (e.g., cleanse) and condition the spent dialysate in a continuous fluid flow loop to recycle the spent dialysate. Example dialysis systems with which the fluid conditioning system 100 can be fluidly communicated include hemodialysis (HD) systems, peritoneal dialysis (PD) systems, hemofiltration (HF), hemodiafiltration (HDF) and other related systems.

The fluid conditioning system 100 includes a housing 101 that contains or supports components of the fluid conditioning system 100, a fluid cassette 102 that includes multiple fluid lines defining various fluid pathways, two relatively high capacity pumps 103 that can circulate fluid within the fluid lines of the fluid cassette 102, and two relatively low capacity pumps 104 that can deliver (e.g., infuse) conditioning agents into the fluid circulating within the fluid lines of the fluid cassette 102. The fluid conditioning system 100 has a compact footprint that facilitates lifting and transport of the fluid conditioning system 100. For example, the fluid conditioning system 100 typically has a length of about 30 cm to about 50 cm, a width of about 30 cm to about 50 cm, a height of about 30 cm to about 50 cm, and a weight of about 15 kg to about 20 kg.

The housing 101 includes left and right side panels 105, 106, handles 107 positioned along the side panels 105, 106 for carrying the fluid conditioning system 100, a door assembly 108 that can be opened and closed to insert a heater bag, a front panel 109 to which the door assembly 108 is secured, rear and bottom panels 110, 111 that further enclose the interior components, an upper panel 112 that supports the fluid cassette 102 and the pumps 103, 104, and a cover 113 that protects the fluid cassette 102 and the pumps 103, 104. Example materials from which the exterior panels of the housing 101 may be made include plastics, such as acrylonitrile butadiene styrene (ABS) and polycarbonate blends, among others.

The cover 113 is typically made of ABS or polycarbonate and is transparent or translucent to allow visualization of the fluid cassette 102 and the pumps 103, 104. The cover 113 can be pivoted at a rear hinge 114 disposed along the upper panel 112 to open or close the cover 113. The upper panel 112 carries two latches 115 that can be closed upon a front edge 116 of the cover 113 to secure the cover 113 in a closed position. The latches 115 can also be pulled up and apart from the cover 113 to release the cover 113 from the closed position for accessing the fluid cassette 102 and the pumps 103, 104.

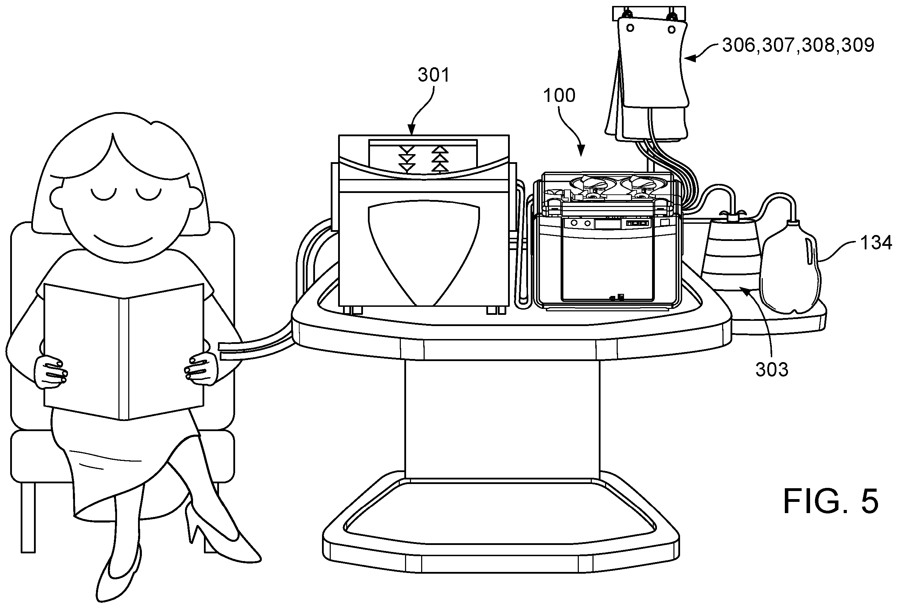

The fluid cassette 102 also includes additional fluid lines that extend from the fluid cassette 102 to various fluid containers, as illustrated in FIG. 5.

FIG. 5 illustrates an example setup of the fluid conditioning system 100. Example types of dialysis systems that may be coupled to the fluid conditioning system 100 include HD systems, PD systems, HF systems, and HDF systems. For example, in addition to the components discussed above with respect to FIGS. 1-4, the fluid conditioning system 100 also includes a sorbent cartridge 303 for filtering tap water to provide purified water suitable for creating dialysate and for cleansing dialysate exiting the dialysis system 301, a bag 306 for containing an electrolyte solution, a bag 307 for containing a salt-dextrose (SD) solution, a bag 308 for containing dilution water (DW), and a bag 309 for containing a bicarbonate (BC) solution.

The bags 306, 307, 309 are pre-loaded with appropriate amounts of dry chemicals that can be dissolved in water to produce the electrolyte solution, the salt-dextrose solution, and the bicarbonate solution. Each bag 306, 307, 309 includes a nozzle that is designed to increase a velocity of a fluid flow entering the bag 306, 307, 309 and to create turbulence needed for adequate mixing and dissolution of the dry chemicals in water.

Table 1 lists approximate capacities of the various fluid-containing components of the fluid conditioning system 100.

**Table 1: Capacities of fluid-containing components of the fluid conditioning system 100.**

| **Component** | **Capacity (mL)** |
|---|---|
| Electrolyte Bag (306) | 500 |
| Salt/Dextrose Bag (307) | 160 |
| Dilution Water Bag (308) | 4000 |
| Bicarbonate Bag (309) | 1000 |

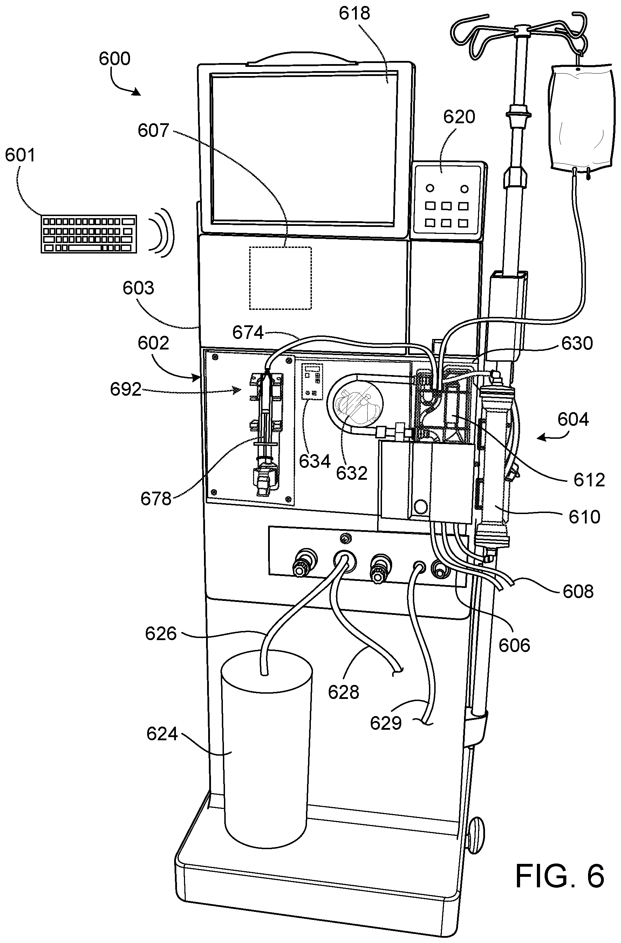

FIG. 6 shows a HD system 600 configured to wirelessly communicate with other medical devices. The HD system 600 includes a HD machine 602 connected to a disposable blood component set 604 that partially forms a blood circuit. The operator can manage and control treatment parameters of the HD system 600 using an external wireless keyboard 601. During HD treatment, an operator connects arterial and venous patient lines 606, 608 of the blood component set 604 to a patient. The blood component set 604 includes an air release device 612, which contains a self-sealing vent assembly that allows air but does not allow liquid to pass. As a result, if blood passing through the blood circuit during treatment contains air, the air release device 612 will vent the air to atmosphere.

The blood component set 604 is secured to a module 630 attached to the front of the HD machine 602. The module 630 includes the blood pump 632 capable of circulating blood through the blood circuit. The module 630 also includes various other instruments capable of monitoring the blood flowing through the blood circuit. The module 630 includes a door that when closed, as shown in FIG. 6, cooperates with the front face of the module 630 to form a compartment sized and shaped to receive the blood component set 604. In the closed position, the door presses certain blood components of the blood component set 604 against corresponding instruments exposed on the front face of the module 630.

The operator uses a blood pump module 634 to operate the blood pump 632. The blood pump module 634 includes a display window, a start/stop key, an up key, a down key, a level adjust key, and an arterial pressure port. The display window displays the blood flow rate setting during blood pump operation. The start/stop key starts and stops the blood pump 632. The up and down keys increase and decrease the speed of the blood pump 632. The level adjust key raises a level of fluid in an arterial drip chamber.

The HD machine 602 further includes a dialysate circuit formed by the dialyzer 610 various other dialysate components and dialysate lines connected to the HD machine 602. Many of these dialysate components and dialysate lines are inside the housing 603 of the HD machine 602 and are thus not visible in FIG. 6. During treatment, while the blood pump 632 circulates blood through the blood circuit, dialysate pumps (not shown) circulate dialysate through the dialysate circuit.

A dialysate container 624 is connected to the HD machine 602 via a dialysate supply line 626. A drain line 628 and an ultrafiltration line 629 also extend from the HD machine 602. The dialysate supply line 626, the drain line 628, and the ultrafiltration line 629 are fluidly connected to the various dialysate components and dialysate lines inside the housing 603 of the HD machine 602 that form part of the dialysate circuit. During HD, the dialysate supply line 626 carries fresh dialysate from the dialysate container 624 to the portion of the dialysate circuit located inside the HD machine 602. As noted above, the fresh dialysate is circulated through various dialysate lines and dialysate components, including the dialyzer 610, that form the dialysate circuit. As will be described below, as the dialysate passes through the dialyzer 610, it collects toxins from the patient's blood. The resulting spent dialysate is carried from the dialysate circuit to a drain via the drain line 628. When ultrafiltration is performed during treatment, a combination of spent dialysate (described below) and excess fluid drawn from the patient is carried to the drain via the ultrafiltration line 629.

The dialyzer 610 serves as a filter for the patient's blood. The dialysate passes through the dialyzer 610 along with the blood, as described above. A semi-permeable structure (e.g., a semi-permeable membrane and/or semi-permeable microtubes) within the dialyzer 610 separates blood and dialysate passing through the dialyzer 610. This arrangement allows the dialysate to collect toxins from the patient's blood. The filtered blood exiting the dialyzer 610 is returned to the patient. The dialysate exiting the dialyzer 610 includes toxins removed from the blood and is commonly referred to as "spent dialysate." The spent dialysate is routed from the dialyzer 610 to a drain.

A drug pump 692 also extends from the front of the HD machine 602. The drug pump 692 is a syringe pump that includes a clamping mechanism configured to retain a syringe 678 of the blood component set 604. The drug pump 692 also includes a stepper motor configured to move the plunger of the syringe 678 along the axis of the syringe 678. A shaft of the stepper motor is secured to the plunger in a manner such that when the stepper motor is operated in a first direction, the shaft forces the plunger into the syringe, and when operated in a second direction, the shaft pulls the plunger out of the syringe 678. The drug pump 692 can thus be used to inject a liquid drug (e.g., heparin) from the syringe 678 into the blood circuit via a drug delivery line 674 during use, or to draw liquid from the blood circuit into the syringe 678 via the drug delivery line 674 during use.

The HD machine 602 includes a user interface with input devices such as a touch screen 618 and a control panel 620. The touch screen 618 and the control panel 620 allow the operator to input various different treatment parameters to the HD machine 602 and to otherwise control the HD machine 602. The touch screen 618 displays information to the operator of the HD system 600. The touch screen 618 can also indicate whether a peripheral or accessory device, such as the keyboard 601, is connected to the HD machine 602. The keyboard 601 is a wireless keyboard that connects to the HD machine 602 by communicating directly or indirectly with a communication system 607 in the HD machine 602. During treatment, the keyboard 601 and other peripheral devices can be used to control, monitor, and determine treatment parameters and variables.

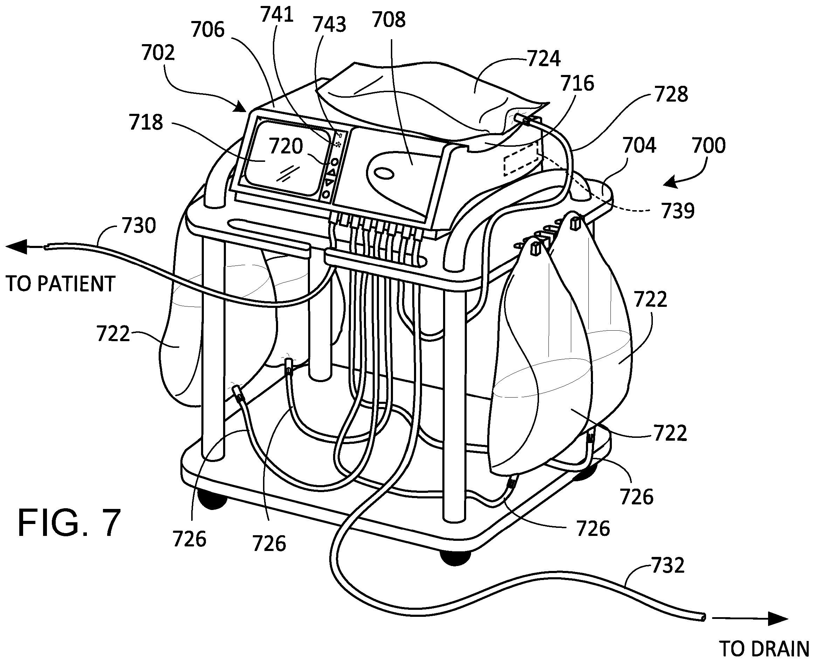

FIG. 7 shows a PD system 700 that includes a PD machine (also referred to as a PD cycler) 702 seated on a cart 704. The PD machine 702 includes a housing 706, a door 708, and a cassette interface that contacts a disposable PD cassette when the cassette is disposed within a cassette compartment formed between the cassette interface and the closed door 708. A heater tray 716 is positioned on top of the housing 706. The heater tray 716 is sized and shaped to accommodate a bag of dialysate (e.g., a 5 liter bag of dialysate). The PD machine 702 also includes a user interface such as a touch screen display 718 and additional control buttons 720 that can be operated by a user (e.g., a caregiver or a patient) to allow, for example, set up, initiation, and/or termination of a PD treatment.

Dialysate bags 722 are suspended from fingers on the sides of the cart 704, and a heater bag 724 is positioned in the heater tray 716. The dialysate bags 722 and the heater bag 724 are connected to the cassette via dialysate bag lines 726 and a heater bag line 728, respectively. The dialysate bag lines 726 can be used to pass dialysate from dialysate bags 722 to the cassette during use, and the heater bag line 728 can be used to pass dialysate back and forth between the cassette and the heater bag 724 during use. In addition, a patient line 730 and a drain line 732 are connected to the cassette. The patient line 730 can be connected to a patient's abdomen via a catheter and can be used to pass dialysate back and forth between the cassette and the patient's peritoneal cavity during use. The drain line 732 can be connected to a drain or drain receptacle and can be used to pass dialysate from the cassette to the drain or drain receptacle during use.

The PD machine 702 also includes a control unit 739 (e.g., a processor), a speaker 741, and a microphone 743. The control unit 739 can receive signals from and transmit signals to the touch screen display 718, the control panel 720, the speaker 741, the microphone 743, and the various other components of the PD system 700. The PD machine 702 can receive audio input (e.g., spoken commands) through the microphone 743 and provide audio output (e.g., spoken alarms, alerts, and instructions) through the speaker 741. The control unit 739 can control the operating parameters of the PD machine 702, for example, based in part on the audio input and output. In some implementations, the control unit 739 is an NPC823 PowerPC device manufactured by Motorola, Inc.

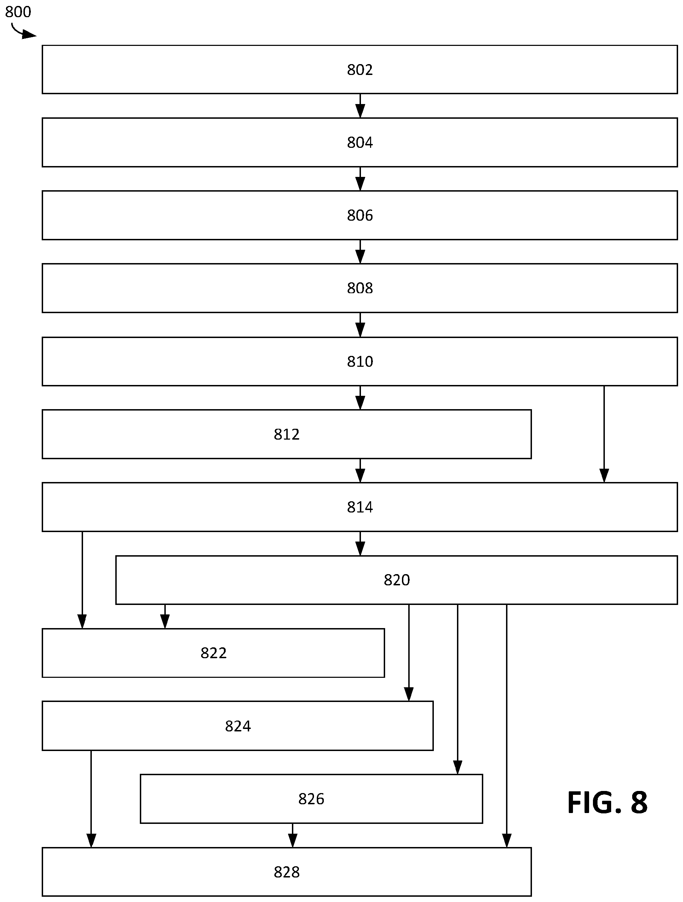

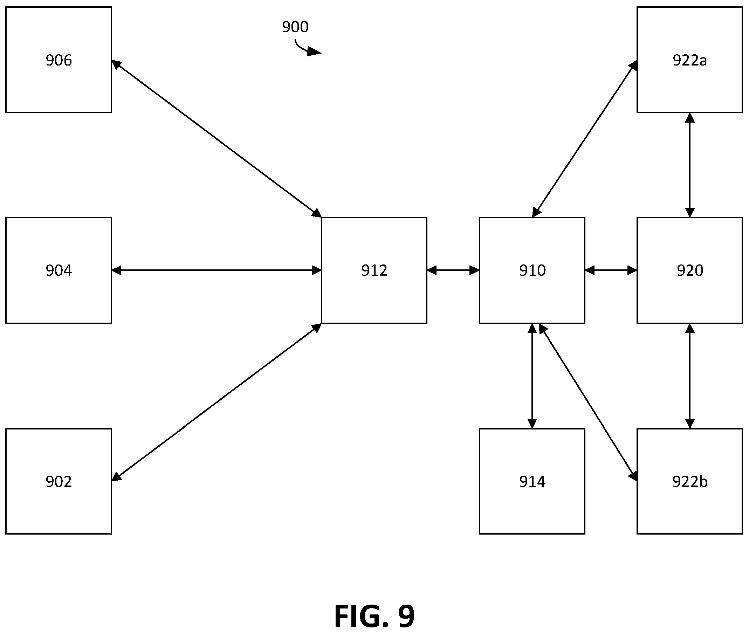

FIG. 8 is an exemplary method for requesting use of a dialysis machine in a dialysis sharing network (800). Using a data processor, a dialysis hub receives a request to use the dialysis machine (e.g., HD machine or PD machine) from a patient (802). In some implementations, the dialysis hub receives the request from the patient's client device (such as a smartphone, tablet, or personal computer). In some implementations, the request is sent from the client device using a mobile application that is connected to a dialysis hub via a network, as illustrated in FIG. 9.

The dialysis hub transmits a list of available dialysis machines to the patient (804). In some implementations, the list of available dialysis machines can be sent to and displayed on the patient's client device (e.g., smartphone, tablet, personal computer). For example, the patient can receive a list of available dialysis machines on their smartphone, or view the list of available dialysis machines on a mobile application. In some implementations, the list of available dialysis machines includes details of the location, hours of operation, availability, price to use, crowdsourced patient reviews, information about and description of the dialysis machine.

In some implementations, the patient receives the list of available dialysis machines and then filters out dialysis machines off the list based on the patient's criteria and preferences (e.g., location and distance, price, reviews, time and date of scheduling availability, etc.). For example, the patient can receive the list of available dialysis machines from the dialysis hub and filter out dialysis machines that which are located too far from the patient's home, and/or which are unavailable on weekends.

**In** some implementations, prior to the dialysis hub receiving the request to use the dialysis machine from the patient, the patient provides filters based on the patient's criteria and preferences. For example, the dialysis hub receives the request to use a dialysis machine that is within a certain radius of the patient's home (e.g., the patient is only willing to travel 10 miles). **In** another example, the dialysis hub receives the request to use a dialysis machine that previous users have given positive reviews for, e.g., 4 out of 5 stars or higher. **In** another example, the dialysis hub receives the request to use a dialysis machine that is available only on weekdays after 5 p.m. when the patient leaves their work.

The dialysis hub receives a request to reserve use of a dialysis machine from the list of dialysis machines (806). In some implementations, the patient transmits the request to reserve use of a dialysis machine that satisfies the patient's criteria and preferences. For example, the patient selects the dialysis machine that is within 10 miles of the patient's home, is available after 5 p.m. and/or on weekends when the patient is not at work, and has sufficient patient reviews so the patient is comfortable with the quality of service they are expected to receive from the dialysis machine provider. The patient transmits the request (e.g., using the client device such as a smartphone) and the request is received by the dialysis hub.

In some implementations, the dialysis hub receives a request to reserve use of the dialysis machine for recurring appointments, e.g., receiving a reservation to use the dialysis machine is rescheduled at regular intervals. For example, many patients require periodic rounds of dialysis, so the request to reserve use of the dialysis machine can specify use of the particular dialysis machine every Tuesday and Thursday for a month, or for three times a week. In some implementations, the dialysis hub can suggest to the patient to reserve the dialysis machine for periodic dialysis treatments. For example, if the patient requests use of the dialysis machine on a Saturday, the dialysis hub can suggest to the patient to request use of the same dialysis machine the following Saturdays as well. The dialysis hub transmits a reservation to use the dialysis machine (808). In some implementations, the dialysis hub transmits to the patient a reservation to use the dialysis machine (e.g., confirmation that the request to reserve use of the dialysis machine was approved). For example, the reservation could be transmitted to the patient's client device as a message (e.g., textual, aural, and/or visual message on the patient's smartphone) that the dialysis machine they requested a reservation for has been reserved for them, with information about the time, date, and location of the dialysis appointment, among other things (e.g., previous patient reviews) (810).

In another implementation, the dialysis hub transmits the reservation to use the dialysis machine to the provider of the dialysis machine (e.g., the person who owns the dialysis machine or the person who operates the dialysis machine). For example, the reservation is sent to the provider of the dialysis machine to allow them to schedule the reservation and prepare for the patient's arrival.

In another implementation, the dialysis hub transmits the reservation to use the dialysis machine to the patient's doctor or physician. For example, the patient's doctor can be informed of their patient's medical care in order to track the patient's diagnosis.

In some scenarios, an alternative dialysis machine may be necessary. For example, the patient may not be able to travel to the location of the reserved dialysis machine, or the dialysis machine may require repairs or maintenance rendering it inoperable. In some implementations, the dialysis hub transmits an alternate reservation to reserve use of a dialysis machine from the list of available dialysis machines (812). The alternate reservation to use the dialysis machine can come from the list of available dialysis machines. For example, the alternate reservation can be the same dialysis machine, but at a different time than the original dialysis appointment. In another example, the alternate reservation can be a different dialysis machine available at or near the same and at or near the same location as the original dialysis appointment.

Prior to dialyzing, the dialysis hub receives a confirmation that the patient has been cannulated (814). Confirmation can be, for example, pressing a button on the dialysis machine and/or the client device confirming cannulation was successful. In another example, confirmation is received using a microphone (e.g., the patient, nurse, or doctor provides an oral confirmation). In another example, confirmation is done using cameras and/or sensors (e.g., a camera records and/or detects the cannulation).

In an embodiment, the patient can be cannulated by a nurse, doctor, or healthcare provider co-located with the dialysis machine. For example, a nurse employed at the dialysis machine location performs the cannulation. In another embodiment, the patient receives instructions from the dialysis hub on how to self-cannulate. For example, the dialysis hub can send to the client device instructions on how to cannulate. In another example, the instructions (or a home training nurse) can provide information on how to self-cannulate using known methods such as the "buttonhole" and/or "rope ladder" methods for cannulation. In another embodiment, the patient can self-cannulate using a needle applicator, e.g., a spring-loaded applicator or plunger-loaded applicator (see, e.g., Dexcom G4 PLATINUM Sensor Applicator https://www.dexcom.com/sites/dexcom.com/files/cgm-education/files/LBL-012207-Rev04-Quick-Start-Guide-G4-PLATINUM-Pro-US.pdt).

Before, during, and/or after the performance of dialysis (820), the dialysis hub can detect the problem and automatically provide emergency support (822). In some implementations, the dialysis hub provides emergency support for situations where the patient experiences difficulties or problems in cannulating by contacting and communicating with emergency personnel (e.g., emergency medical technicians). For example, if too much time has passed between signaling the start of the cannulation process (e.g., by pressing a button on the dialysis machine and/or the client device indicating cannulation is going to be performed) and confirmation of the cannulation, the dialysis hub provides emergency support by contacting and communicating with emergency personnel. For example, the emergency support the dialysis hub can provide includes but is not limited to troubleshooting information for cannulation and contacting emergency personnel (e.g., emergency medical technicians, nurses, doctors).

In some implementations, the dialysis hub obtains data (824). For example, the dialysis hub obtains crowdsourced patient reviews of the dialysis machine, patient reviews of the provider of the dialysis machine, and amenities and services offered during dialysis. Further, the dialysis hub obtains diagnostic data in the form of real-time data and historical data. This allows the patient (and their doctor) to determine the progress of the patient's current condition, and compare it to the patient's past conditions.

In some implementations, obtaining data refers to collecting dialysis machine and/or dialysis machine provider information. For example, data can be obtained that indicates the dialysis machine unreliable and constantly requires repairs, or that the provider of the dialysis machine does not follow proper dialysis procedures (e.g., holds themselves out as knowing how to cannulate but consistently makes mistakes, or does not keep the environment clean).

In some implementations, the dialysis hub can provide remote monitoring of the dialysis machine (826). For example, the dialysis hub can provide the patient with the information regarding the progress of the dialysis (e.g., the patient can see on their client device that they are halfway through the dialysis appointment). The dialysis hub can provide the patient's doctor with real-time data (e.g., so the doctor can monitor the patient's dialysis without having to be there for the entire duration of the dialysis). The dialysis information provided by remote monitoring can be displayed, for example, on the client device.

In some implementations, the remote monitoring can provide a dialysis machine technician information regarding the dialysis machine's hardware and software. For example, if the dialysis machine malfunctions (e.g., a motor or pump is broken) then a technician can be dispatched immediately to resolve the problem. In addition, historical data can be obtained from remote monitoring. For example, if a pump on the dialysis machine consistently breaks after 100 uses, then the dialysis hub can predict and prevent malfunctions by dispatching a technician to repair the dialysis machine.

The dialysis hub provides risk reduction (828). If the crowdsourced patient data about the dialysis machines indicates that a particular machine is prone to breakdowns or malfunctions, the dialysis hub removes the particular dialysis machine from the list of available machines until the particular machine is repaired or replaced. In an example, risk reduction includes providing providers of dialysis machines with supplies required for dialysis, and determining when the provider is running low on supplies. In another example, crowdsourced patient data about a particular provider of a dialysis machine can reduce risk of performing dialysis in a location that is not conducive or otherwise unfit for safe dialysis (e.g., the location is unsanitary, the provider consistently fails to satisfy the needs of patients with regards to scheduling or comfort, etc.).

In some implementations, remote monitoring of the patient and/or dialysis machine can provide risk reduction. For example, a patient's vitals may be remotely monitored by a nurse or doctor during dialysis, and any alarming changes (say, for example, a rapid rise in the patient's heartrate and blood pressure) may be addressed by the nurse or doctor immediately to prevent further harm to the patient. In another example, remote monitoring of the dialysis machine can provide a technician information to alert them of any dialysis machine malfunctions or breakdowns.

FIG. 9 is an exemplary system for a dialysis sharing network system 900. The system includes a data processor 910, a network 912, a dialysis hub 920, and dialysis machines 922a-b. Additionally, the system can include a client device 902, a physician terminal 904, an emergency terminal 906, and a database 914. The system 900 includes the data processor 910. In an embodiment, the data processor 910 is a server (e.g., a cloud-based processor, computer, or server). For example, the data processor 910 is a cloud-based processor that receives, over the network 912 and from the client device 902, a request to use a dialysis machine 922a-b, and transmits the request to the dialysis hub 920.

The system 900 includes the client device 902. In an embodiment, the client device 902 is a smartphone, tablet, and/or personal computer. In an embodiment, the client device 902 includes a mobile application configured to communicate with the dialysis hub 920 using the data processor 910 and over the network 912. For example, the dialysis hub 920 can receive and transmit information to and from the client device 902 using the data processor 910 and over the network 912.

In an embodiment, the dialysis machine 922a is a primary dialysis machine (e.g., the dialysis machine that the patient selects for their dialysis). The dialysis machines 922b are alternative dialysis machines in the network of dialysis machines (e.g., one or more dialysis machines at one or more locations).

In an embodiment, the client device 902 can communicate with the database 914 using the data processor 910 and over the network 912. In an embodiment, the database 914 is a server (e.g., a cloud based processor, computer, or server). The client device 902 can communicate with the dialysis machines 922a-b using the data processor 910 (and/or the dialysis hub 920 using the data processor 910) and over the network 912. For example, the client device 902 can receive and transmit from the database 914 patient reviews about the dialysis machines 922a-b and the providers of the dialysis machines (e.g., whether the patient had a good experience using the particular dialysis machine).

In an embodiment, the client device 902 communicates with the physician terminal 904. For example, the client device 902 can send dialysis information received from the database 914, the dialysis hub 920, and/or the dialysis machines 922a-b to their doctor at the physician terminal 904 via the network 912. For example, this can be done by pressing a button on the client device 902 that indicates the patient would like to share the dialysis results directly to the doctor.

In an embodiment, the client device 902 communicates with the emergency terminal 906. For example, the client device 902 can contact emergency medical personnel or medical assistants (e.g., nurses trained in dialysis procedures) via the network 912. For example, this can be done by pressing a button on the client device indicating the patient would like help or assistance. In another example, the patient can be required to periodically confirm or check-in to indicate that they do not need help, and if a confirmation is not sent, emergency medical personnel are automatically dispatched to the patient's location.

The dialysis hub 920 communicates with the dialysis machines 922a-b. The dialysis hub 920 receives information about the dialysis machines 922a-b including availability, repair and maintenance status, patient reviews associated with the dialysis machine and its provider and location. In another example, the dialysis hub 920 transmits information to the dialysis machines 922a-b (e.g., a reservation to use the machine).

In an embodiment, the dialysis hub 920 communicates with the database 914. For example, the dialysis hub 920 can transmit to the database 914 the crowd-sourced patient reviews of the dialysis machines 922a-b and the providers of the dialysis machines. In another example, the dialysis hub 920 can transmit to the database 914 diagnostic data regarding the dialysis machines 922a-b (e.g., machine repair or maintenance status).

In an embodiment, the dialysis hub 920 communicates with the physician terminal 904. For example, the dialysis hub 920 transmits dialysis information from the dialysis machines 922a-b to a doctor at the physician terminal 904 for remote monitoring of the patient's dialysis. In another example, the dialysis hub transmits to the doctor at the physician terminal diagnostic information related to the patient's dialysis.

In an embodiment, the dialysis hub 920 communicates with the emergency terminal 906. For example, the dialysis hub 920 can detect when a patient is unable to cannulate and can contact the emergency terminal 906 to dispatch a nurse to aid the patient. In another example, the dialysis hub, communicating with the dialysis machines 922a-b, can detect dangerous fluctuations in a patient's vitals (e.g., blood pressure) and dispatch emergency medical technicians to assist the patient.

Implementations of the subject matter and the functional operations described above can be implemented in other types of digital electronic circuitry, or in computer software, firmware, or hardware, including the structures disclosed in this specification and their structural equivalents, or in combinations of one or more of them. Implementations of the subject matter described in this specification can be implemented as one or more computer program products, i.e., one or more modules of computer program instructions encoded on a tangible program carrier, for example a computerreadable medium, for execution by, or to control the operation of, a processing system. The computer readable medium can be a machine readable storage device, a machine readable storage substrate, a memory device, a composition of matter effecting a machine readable propagated signal, or a combination of one or more of them.

The term "computer system" may encompass all apparatus, devices, and machines for processing data, including by way of example a programmable processor, a computer, or multiple processors or computers. A processing system can include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, executable logic, or code) can be written in any form of programming language, including compiled or interpreted languages, or declarative or procedural languages, and it can be deployed in any form, including as a standalone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

Computer readable media suitable for storing computer program instructions and data include all forms of non-volatile or volatile memory, media and memory devices, including by way of example semiconductor memory devices, e.g., EPROM, EEPROM, and flash memory devices; magnetic disks, e.g., internal hard disks or removable disks or magnetic tapes; magneto optical disks; and CD-ROM and DVD-ROM disks. The processor and the memory can be supplemented by, or incorporated in, special purpose logic circuitry. The components of the system can be interconnected by any form or medium of digital data communication, e.g., a communication network. Examples of communication networks include a local area network ("LAN") and a wide area network ("WAN"), e.g., the Internet.

A number of embodiments have been described in detail above.

Other embodiments are within the scope of the following claims.

## Claims

1. A method executed by a dialysis hub system (900), the method comprising:
receiving, from a plurality of dialysis machines (922) and using a data processor, information about the plurality of dialysis machines (922), the information including availability, repair and maintenance status, patient reviews associated with the dialysis machines and provider and location of the dialysis machines;
receiving (802), using the data processor, a request to use a dialysis machine;
filtering a list of available dialysis machines according to time, date, and/or location;
if the patient reviews indicate that a particular of the dialysis machines is prone to breakdowns or malfunctions, removing (828) the particular dialysis machine from the list of available dialysis machines until the particular dialysis machine is repaired or replaced;
transmitting (804), using the data processor, the list of available dialysis machines;
receiving (806), using the data processor, a request to reserve use of a first dialysis machine from among dialysis machines in the list of available dialysis machines; and
transmitting (808), using the data processor, a reservation to use the first dialysis machine.

2. The method of claim 1, further comprising:
transmitting (812), using the data processor, an alternate reservation to use a second dialysis machine from the list of available dialysis machines.

3. The method of any of the preceding claims, further comprising:
transmitting, using the data processor, instructions for self-cannulation.

4. The method of any of the preceding claims, further comprising:
receiving dialysis information from the dialysis machines; and
transmitting, using the data processor, the dialysis information to a client device (902).

5. The method of any of the preceding claims, further comprising:
communicating, using the data processor, with emergency personnel.

6. The method of any of the preceding claims, further comprising:
receiving (824), using the data processor, diagnostic data from the dialysis machine.

7. The method of any of the preceding claims, wherein the dialysis hub system (900) is configured to communicate between multiple parties including multiple patients, multiple providers of dialysis machines, multiple medical personnel, and multiple emergency personnel.

8. A dialysis network system (900) comprising:
a dialysis hub (920), wherein:
the dialysis hub is configured to receive information about a plurality of dialysis machines (922), the information including availability, repair and maintenance status, patient reviews associated with the dialysis machines and provider and location of the dialysis machines;
the dialysis hub is configured to receive (802) a request to use a dialysis machine, wherein the request is received from a client device using a data processor and over a network;
the dialysis hub is configured to filter a list of available dialysis machines according to time, date, and/or location;
if the patient reviews indicate that a particular of the dialysis machines is prone to breakdowns or malfunctions, the dialysis hub is configured to remove (828) the particular dialysis machine from the list of available dialysis machines until the particular dialysis machine is repaired or replaced;
the dialysis hub is configured to transmit (804) the list of available dialysis machines, wherein the list of available dialysis machines is transmitted to the client device using the data processor and over the network;
the dialysis hub is configured to receive (806) a request to reserve use of a first dialysis machine from among dialysis machines in the list of available dialysis machines, wherein the request to reserve use of the first dialysis machine is received from the client device using the data processor and over the network; and
the dialysis hub is configured to transmit (808) a reservation to use the first dialysis machine, wherein the reservation to use the first dialysis machine is transmitted to the client device using the data processor and over the network.

9. The system of claim 8, wherein the dialysis hub is further configured to transmit dialysis data to the client device (902) or to a physician terminal (904).

10. The system of any of claims 8 through 9, wherein the dialysis hub is further configured to communicate with an emergency terminal (906).

11. The system of any of claims 8 through 10, wherein the dialysis hub is further configured to transmit information from a database (914) to the client device (902).

12. The system of any of claims 8 through 11, wherein the dialysis hub is further configured to update a database (914) with information from the client device (902).

13. The system of any of claims 8 through 12, wherein the dialysis hub is configured to communicate between multiple parties including multiple patients, multiple providers of dialysis machines, multiple medical personnel, and multiple emergency personnel.

14. A non-transitory computer storage medium encoded with a computer program, the computer program comprising instructions that when executed by data processing apparatus cause the data processing apparatus to perform operations comprising the method of any one of claims 1 to 7.

## Patentansprüche

1. Verfahren, das durch ein Dialyse-Hub-System (900) ausgeführt wird, wobei das Verfahren Folgendes umfasst:
Empfangen von Informationen über die Vielzahl von Dialysegeräten (922) von einer Vielzahl von Dialysegeräten (922) und unter Verwendung eines Datenprozessors, wobei die Informationen Verfügbarkeit, Reparatur- und Wartungsstatus, Patientenberichte, die den Dialysegeräten zugeordnet sind, und Anbieter und Standort der Dialysegeräte beinhalten;
Empfangen (802) einer Anforderung, ein Dialysegerät zu verwenden, unter Verwendung des Datenprozessors;
Filtern einer Liste verfügbarer Dialysegeräte nach Zeit, Datum und/oder Standort;
falls die Patientenberichte angeben, dass ein bestimmtes Dialysegerät anfällig für Ausfälle oder Störungen ist, Entfernen (828) des bestimmten Dialysegeräts aus der Liste der verfügbaren Dialysegeräte, bis das bestimmte Dialysegerät repariert oder ausgetauscht wurde;
Übermitteln (804) der Liste der verfügbaren Dialysegeräte unter Verwendung des Datenprozessors;
Empfangen (806) einer Anforderung, die Verwendung eines ersten Dialysegeräts unter Dialysegeräten in der Liste der verfügbaren Dialysegeräte zu reservieren, unter Verwendung des Datenprozessors; und
Übertragen (808) einer Reservierung zur Verwendung des ersten Dialysegeräts unter Verwendung des Datenprozessors.

2. Verfahren nach Anspruch 1, ferner Folgendes umfassend: Übertragen (812) einer alternativen Reservierung zur Verwendung eines zweiten Dialysegeräts aus der Liste der verfügbaren Dialysegeräte unter Verwendung des Datenprozessors.

3. Verfahren nach einem der vorstehenden Ansprüche, ferner Folgendes umfassend:
Übertragen von Anweisungen zur Selbstkanülierung unter Verwendung des Datenprozessors.

4. Verfahren nach einem der vorstehenden Ansprüche, ferner Folgendes umfassend:
Empfangen von Dialyseinformationen von den Dialysegeräten; und
Übertragen der Dialyseinformationen an eine Client-Vorrichtung (902) unter Verwendung des Datenprozessors.

5. Verfahren nach einem der vorstehenden Ansprüche, ferner Folgendes umfassend:
Kommunizieren mit Notfallpersonen unter Verwendung des Datenprozessors.

6. Verfahren nach einem der vorstehenden Ansprüche, ferner Folgendes umfassend:
Empfangen (824) von Diagnosedaten von dem Dialysegerät unter Verwendung des Datenprozessors.

7. Verfahren nach einem der vorhergehenden Ansprüche, wobei das Dialyse-Hub-System (900) dazu ausgelegt ist, zwischen mehreren Beteiligten, einschließlich mehrerer Patienten, mehrerer Anbieter von Dialysegeräten, mehrerer medizinischer Mitarbeiter und mehrerer Notfallpersonen, zu kommunizieren.

8. Dialysenetzsystem (900), das Folgendes umfasst:
einen Dialyse-Hub (920), wobei:
der Dialyse-Hub zum Empfangen von Informationen über eine Vielzahl von Dialysegeräten (922) ausgelegt ist, wobei die Informationen Verfügbarkeit, Reparatur- und Wartungsstatus, Patientenberichte, die den Dialysegeräten zugeordnet sind, und Anbieter und Standort der Dialysegeräte beinhalten;
der Dialyse-Hub dazu ausgelegt ist, eine Anforderung zur Verwendung eines Dialysegeräts zu empfangen (802), wobei die Anforderung von einer Client-Vorrichtung unter Verwendung eines Datenprozessors und über ein Netz empfangen wird;
der Dialyse-Hub dazu ausgelegt ist, eine Liste verfügbarer Dialysegeräte nach Zeit, Datum und/oder Standort zu filtern;
wobei der Dialyse-Hub dazu ausgelegt ist, das bestimmte Dialysegerät aus der Liste der verfügbaren Dialysegeräte zu entfernen (828), bis das bestimmte Dialysegerät repariert oder ausgetauscht wird, falls die Patientenberichte darauf hinweisen, dass ein bestimmtes der Dialysegeräte anfällig für Ausfälle oder Störungen ist;
der Dialyse-Hub dazu ausgelegt ist, die Liste verfügbarer Dialysegeräte zu übertragen (804), wobei die Liste verfügbarer Dialysegeräte unter Verwendung des Datenprozessors und über das Netz an die Client-Vorrichtung übertragen wird;
der Dialyse-Hub dazu ausgelegt ist, eine Anforderung zu empfangen (806), die Verwendung eines ersten Dialysegeräts unter Dialysegeräten in der Liste verfügbarer Dialysegeräte zu reservieren, wobei die Anforderung, die Verwendung des ersten Dialysegeräts zu reservieren, von der Client-Vorrichtung unter Verwendung des Datenprozessors und über das Netz empfangen wird; und
der Dialyse-Hub dazu ausgelegt ist, eine Reservierung zur Verwendung des ersten Dialysegeräts zu übertragen (808), wobei die Reservierung zur Verwendung des ersten Dialysegeräts unter Verwendung des Datenprozessors und über das Netz an die Client-Vorrichtung übertragen wird.

9. System nach Anspruch 8, wobei der Dialyse-Hub ferner dazu ausgelegt ist, Dialysedaten an die Client-Vorrichtung (902) oder an ein Ärzteendgerät (904) zu übertragen.

10. System nach einem der Ansprüche 8 bis 9, wobei der Dialyse-Hub ferner dazu ausgelegt ist, mit einem Notfallendgerät (906) zu kommunizieren.

11. System nach einem der Ansprüche 8 bis 10, wobei der Dialyse-Hub ferner dazu ausgelegt ist, Informationen von einer Datenbank (914) an die Client-Vorrichtung (902) zu übertragen.

12. System nach einem der Ansprüche 8 bis 11, wobei der Dialyse-Hub ferner dazu ausgelegt ist, eine Datenbank (914) mit Informationen von der Client-Vorrichtung (902) zu aktualisieren.

13. System nach einem der Ansprüche 8 bis 12, wobei der Dialyse-Hub dazu ausgelegt ist, zwischen mehreren Beteiligten, einschließlich mehrerer Patienten, mehrerer Anbieter von Dialysegeräten, mehrerer medizinischer Mitarbeiter und mehrerer Notfallpersonen, zu kommunizieren.

14. Nichttransitorisches Computerspeichermedium, das mit einem Computerprogramm codiert ist, wobei das Computerprogramm Anweisungen umfasst, die bei Ausführung durch eine Datenverarbeitungseinrichtung bewirken, dass die Datenverarbeitungseinrichtung Operationen durchführt, die das Verfahren nach einem der Ansprüche 1 bis 7 umfassen.

## Revendications

1. Procédé exécuté par un système de hub de dialyse (900), le procédé comprenant :
la réception, en provenance d'une pluralité de machines de dialyse (922) et au moyen d'un processeur de données, d'informations concernant la pluralité de machines de dialyse (922), les informations comportant la disponibilité, l'état de réparation et de maintenance, les avis de patients associés aux machines de dialyse ainsi que le fournisseur et l'emplacement des machines de dialyse ;
la réception (802), au moyen du processeur de données, d'une demande d'utilisation d'une machine de dialyse ;
le filtrage d'une liste de machines de dialyse disponibles selon l'heure, la date et/ou l'emplacement ;
si les avis de patients indiquent qu'une machine de dialyse particulière parmi les machines de dialyse est sujette à des pannes ou dysfonctionnements, le retrait (828) de la machine de dialyse particulière de la liste de machines de dialyse disponibles jusqu'à ce que la machine de dialyse particulière soit réparée ou remplacée ;
la transmission (804), au moyen du processeur de données, de la liste de machines de dialyse disponibles ;
la réception (806), au moyen du processeur de données, d'une demande de réservation pour l'utilisation d'une première machine de dialyse parmi des machines de dialyse figurant dans la liste de machines de dialyse disponibles ; et
la transmission (808), au moyen du processeur de données, d'une réservation pour l'utilisation de la première machine de dialyse.

2. Procédé de la revendication 1, comprenant en outre : la transmission (812), au moyen du processeur de données, d'une réservation de substitution pour l'utilisation d'une deuxième machine de dialyse parmi la liste de machines de dialyse disponibles.

3. Procédé selon l'une quelconque des revendications précédentes, comprenant en outre :
la transmission, au moyen du processeur de données, d'instructions pour l'auto-canulation.

4. Procédé selon l'une quelconque des revendications précédentes, comprenant en outre :
la réception d'informations de dialyse en provenance des machines de dialyse ; et
la transmission, au moyen du processeur de données, des informations de dialyse à un dispositif client (902).

5. Procédé selon l'une quelconque des revendications précédentes, comprenant en outre :
la communication, au moyen du processeur de données, avec du personnel d'urgence.

6. Procédé selon l'une quelconque des revendications précédentes, comprenant en outre :
la réception (824), au moyen du processeur de données, de données de diagnostic en provenance de la machine de dialyse.

7. Procédé selon l'une quelconque des revendications précédentes, dans lequel le système de hub de dialyse (900) est configuré pour communiquer entre de multiples participants comportant de multiples patients, de multiples fournisseurs de machines de dialyse, de multiples membres du personnel médical et de multiples membres du personnel d'urgence.

8. Système de réseau de dialyse (900) comprenant :
un hub de dialyse (920), dans lequel :
le hub de dialyse est configuré pour recevoir des informations concernant une pluralité de machines de dialyse (922), les informations comportant la disponibilité, l'état de réparation et de maintenance, les avis de patients associés aux machines de dialyse ainsi que le fournisseur et l'emplacement des machines de dialyse ;
le hub de dialyse est configuré pour recevoir (802) une demande d'utilisation d'une machine de dialyse, la demande étant reçue en provenance d'un dispositif client au moyen d'un processeur de données et via un réseau ;
le hub de dialyse est configuré pour filtrer une liste de machines de dialyse disponibles selon l'heure, la date et/ou l'emplacement ;
si les avis de patients indiquent qu'une machine de dialyse particulière parmi les machines de dialyse est sujette à des pannes ou dysfonctionnements, le hub de dialyse est configuré pour retirer (828) la machine de dialyse particulière de la liste de machines de dialyse disponibles jusqu'à ce que la machine de dialyse particulière soit réparée ou remplacée ;
le hub de dialyse est configuré pour transmettre (804) la liste de machines de dialyse disponibles, la liste de machines de dialyse disponibles étant transmise au dispositif client au moyen du processeur de données et via le réseau ;
le hub de dialyse est configuré pour recevoir (806) une demande de réservation pour l'utilisation d'une première machine de dialyse parmi des machines de dialyse figurant dans la liste de machines de dialyse disponibles, la demande de réservation pour l'utilisation de la première machine de dialyse étant reçue en provenance du dispositif client au moyen du processeur de données et via le réseau ; et
le hub de dialyse est configuré pour transmettre (808) une réservation pour l'utilisation de la première machine de dialyse, la réservation pour l'utilisation de la première machine de dialyse étant transmise au dispositif client au moyen du processeur de données et via le réseau.

9. Système selon la revendication 8, dans lequel le hub de dialyse est en outre configuré pour transmettre des données de dialyse au dispositif client (902) ou à un terminal de médecin (904).

10. Système selon l'une quelconque des revendications 8 à 9, dans lequel le hub de dialyse est en outre configuré pour communiquer avec un terminal d'urgence (906).

11. Système selon l'une quelconque des revendications 8 à 10, dans lequel le hub de dialyse est en outre configuré pour transmettre des informations provenant d'une base de données (914) au dispositif client (902).

12. Système selon l'une quelconque des revendications 8 à 11, dans lequel le hub de dialyse est en outre configuré pour mettre à jour une base de données (914) avec des informations en provenance du dispositif client (902).

13. Système selon l'une quelconque des revendications 8 à 12, dans lequel le hub de dialyse est configuré pour communiquer entre de multiples participants comportant de multiples patients, de multiples fournisseurs de machines de dialyse, de multiples membres du personnel médical et de multiples membres du personnel d'urgence.

14. Support de stockage informatique non transitoire codé avec un programme informatique, le programme informatique comprenant des instructions qui, lorsqu'elles sont exécutées par un appareil de traitement de données, amènent l'appareil de traitement de données à réaliser des opérations comprenant le procédé selon l'une quelconque des revendications 1 à 7.
